# EUROPEAN PATENT APPLICATION

(11) **EP 1 972 624 A1**
(43) Date of publication of application: **24.09.2008**
(21) Application number: 07290360.2
(22) Date of filing: 23.03.2007
(51) Int. Cl.: C07D 413/04, C08K 5/34

(54) **Benzoxazinones and their use as ultraviolet light absorbers**

(71) Applicant: Clariant International Ltd., 4132 Muttenz 1 (CH)
(72) Inventor: Pays, Christophe, 68170 Rixheim (FR); Kröhnke, Christoph, 79206 Breisach-Oberrimsingen (DE)

(57) **Abstract**

The present invention relates to a new subclass of UV absorbers based on benzothiophene substituted benzoxazinones which can be used for stabilization of organic substrates, preferably in the protection of organic substrates against the damaging influence of light exposure.

## Description

The present invention relates to a new subclass of UV absorbers based on benzothiophene substituted benzoxazinones which can be used for stabilization of organic substrates, preferably in the protection of organic substrates against the damaging influence of light exposure.

UV absorbers have attracted considerable attention in the field of polymer stabilization. Generally many technical polymers as well as coatings contain UV absorbers of hydroxybenzophenone, benzotriazole, hydroxyphenyltriazine, benzylidenemalonate, cinnamate, oxanilide and other classes. These UV absorbers are more or less suitable in their respective fields with the given absorption criteria.

Benzoxazinones are known UV-absorbers. Most members of this group of compounds absorb within a broad range overlapping both higher wavelengths of UVA (320 to 360 nm) and shorter wavelength of the UVB spectrum (290 to 320 nm).

Desired properties ofbenzoxazinone based UV absorbers are color neutrality, low volatility, high solubility preferably in organic solvents (e.g. for coating applications), high compatibility or miscibility in polymeric substrates, low melting points, high absorption in the UV range, preferably with a red shifted absorption decay as close as possible to the initial wavelength of visible light (400nm). Other requirements for benzoxazinone based UV absorbers are long lifetime, high thermal stability, high durability during storage and non-toxicity. These requirements are basically the same both for the use of benzoxazinone based UV absorbers in technical polymers as well as in coatings. For color neutrality, the absorption of the benzoxazinone based UV absorbers in the visible range should be low.

US 6774232 B discloses benzoxazinone compounds of defined structures used as UV absorbers for polymeric material. These compounds can be also used for engineering plastics such as polycarbonate, polyamides, polyesters as well as for sunscreens, cosmetics and fibers.

US 2004/0192684 A discloses UV absorbers of the benzoxazinone, oxanilide, benzylidene malonate, quinazoline and benzotriazole classes. The disclosure relates to polymer and photographic compositions, stabilized against the deleterious effects of light induced degradation, which comprise the UV light absorbers.

WO 2005/118562 discloses substituted benzoxazinones and their use as UV light absorbers, in particular for organic polymers.

Despite their common applicability, the known UV absorbers suffer often from low compatibility in the corresponding polymeric substrates and from other unfavorable physical properties which limit their usability. There is still a need for UV absorber with broad absorption in the near UV wavelength range (UV-A range), which are also sufficiently thermally stable in order to protect organic substrates efficiently against damaging influences of the environment. Furthermore it is desired to have more efficient UV absorbers allowing dosages at low concentrations.

Surprisingly it has now been found that certain benzothiophene substituted benzoxazinones are efficient in protecting organic substrates against the damaging influences of light during their lifetime.

In the following text "halogen" represents F, Cl, Br and I, preferably F, Cl or Br, more preferably F or Cl, even more preferably Cl; "alkyl" and "alkenyl" represent linear or branched alkyl and alkenyl; if not otherwise stated.

Subject of the invention is a compound of formula (I), 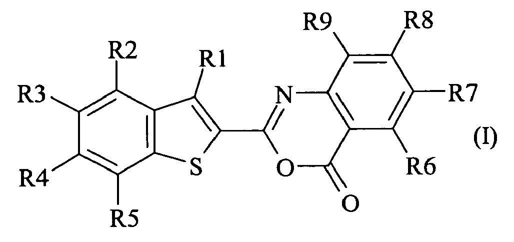 wherein
R1, R2, R3, R4, R5, R6, R7, R8 and R9 are same or different and independently from each other selected from the group consisting of H, CN, halogen, OH,
   C₁₋₁₈ alkyl, the C₁₋₁₈ alkyl being unsubstituted or substituted by C₁₋₁₈ alkyl, C₁₋₈ alkenyl, C₁₋₈ alkoxy, halogen, OH or C₆₋₁₂ aryl,
   C₁₋₁₈ alkenyl, C₃₋₁₈ cycloalkyl, C₃₋₁₈ cycloalkenyl,
   C₆₋₁₂ aryl, the C₆₋₁₂ aryl being unsubstituted or substituted by C₁₋₈ alkyl, C₁₋₈ alkenyl, C₁₋₈ alkoxy, halogen or OH,
   OC₁₋₁₈ alkyl, the C₁₋₁₈ alkyl residue being unsubstituted or substituted by C₁₋₈ alkyl, C₁₋₈ alkenyl, OH, C₆₋₁₂ aryl or NR10R11,
   OC₆₋₁₂ aryl, the C₆₋₁₂ aryl residue being unsubstituted or substituted by C₁₋₈ alkyl, C₁₋₈ alkenyl, OH or NR10R11, and
   OC(O)R12;
R12 is
   C₁₋₁₈ alkyl, the C₁₋₁₈ alkyl being unsubstituted or substituted by C₁₋₈ alkyl, C₁₋₈ alkenyl, OH, C₆₋₁₂ aryl or NR10R11, or R12 is
   C₁₋₁₂ aryl, the C₁₋₁₂ aryl residue being unsubstituted or substituted by C₁₋₈ alkyl, C₁₋₈ alkenyl, OH, or NR10R11;
the R10 and R11 residues are same or different and independently from each other selected from the group consisting of H, C₁₋₈ alkyl, C₁₋₈ alkenyl and C₆₋₁₂ aryl.

Preferably, subject of the invention is a compound of formula (I), wherein
R1 is selected from the group consisting of H, CN, halogen, OH,
   C₁₋₁₈ alkyl, the C₁₋₁₈ alkyl being unsubstituted or substituted by C₁₋₈ alkyl, C₁₋₈ alkenyl, C₁₋₈ alkoxy, halogen, OH or C₆₋₁₂ aryl, C₁₋₁₈ alkenyl, C₃₋₁₈ cycloalkyl, C₃₋₁₈ cycloalkenyl,
   C₆₋₁₂ aryl, the C₆₋₁₂ aryl being unsubstituted or substituted by C₁₋₈ alkyl, C₁₋₈ alkenyl, C₁₋₈ alkoxy, halogen or OH,
   OC₁₋₁₈ alkyl, the C₁₋₁₈ alkyl residue being unsubstituted or substituted by C₁₋₈ alkyl, C₁₋₈ alkenyl, OH, C₆₋₁₂ aryl or NR10R11,
   OC₆₋₁₂ aryl, the C₆₋₁₂ aryl residue being unsubstituted or substituted by C₁₋₈ alkyl, C₁₋₈ alkenyl, OH or NR10R11, and
   OC(O)R12;
R12 is
   C₁₋₁₈ alkyl, the C₁₋₁₈ alkyl being unsubstituted or substituted by C₁₋₈ alkyl, C₁₋₈ alkenyl, OH, C₆₋₁₂ aryl or NR10R11, or R12 is
   C₁₋₁₂ aryl, the C₁₋₁₂ aryl residue being unsubstituted or substituted by C₁₋₈ alkyl, C₁₋₈ alkenyl, OH, or NR10R11;
R2, R4, R5, R6, R7, R8 and R9 are H;
R3 is selected from the group consisting of H, CN, halogen, OH,
   C₁₋₁₈ alkyl, the C₁₋₁₈ alkyl being unsubstituted or substituted by C₁₋₈ alkyl, C₁₋₈ alkenyl, C₁₋₈ alkoxy, halogen or C₆₋₁₂ aryl, and
   OC₁₋₁₈ alkyl, the C₁₋₁₈ alkyl residue being unsubstituted or substituted by C₁₋₈ alkyl or C₆₋₁₂ aryl;
the R10 and R11 residues are same or different and independently from each other selected from the group consisting of H, C₁₋₈ alkyl, C₁₋₈ alkenyl and C₆₋₁₂ aryl.

More preferably, subject of the invention is a compound of formula (I), wherein
R1 is selected from the group consisting of H, Cl,
   C₁₋₁₈ alkyl, the C₁₋₁₈ alkyl being unsubstituted or substituted by C₁₋₈ alkyl,
   OC₁₋₁₈ alkyl, the C₁₋₁₈ alkyl residue being unsubstituted or substituted by C₁₋₈ alkyl, and
   OC(O)R12;
R12 is
   C₁₋₁₈ alkyl, the C₁₋₁₈ alkyl residue being unsubstituted or substituted by C₁₋₈ alkyl, or R12 is
   C₁₋₁₂ aryl, the C₁₋₁₂ aryl residue being unsubstituted or substituted by C₁₋₈ alkyl;
   R2, R4, R5, R6, R7, R8 and R9 are H;
   R3 is H or methyl.

Even more preferably, subject of the invention is a compound of formula (I),
wherein
R1 is selected from the group consisting of Cl, methyl, undecyl, 1-ethylpentyl, O-octyl, O-2-ethylhexyl, O-dodecyl, O-hexadecyl and OC(O)R12;
R2, R4, R5, R6, R7, R8 and R9 are H;
R3 is H or methyl;
R12 is selected from the group consisting of methyl, ethyl, n-propyl, isopropyl, n-butyl, isobutyl, pentyl, isopentyl, hexyl, heptyl, 3-heptyl, octyl, 2-ethylhexyl, phenyl, o-tolyl, m-tolyl and p-tolyl.

Especially preferably, subject of the invention is a compound of a formula selected from the group of formulae (Ia), (Ib), (Ic), (Id), (Ie), (If), (Ig), (Ih), (Ii) and (Ij).

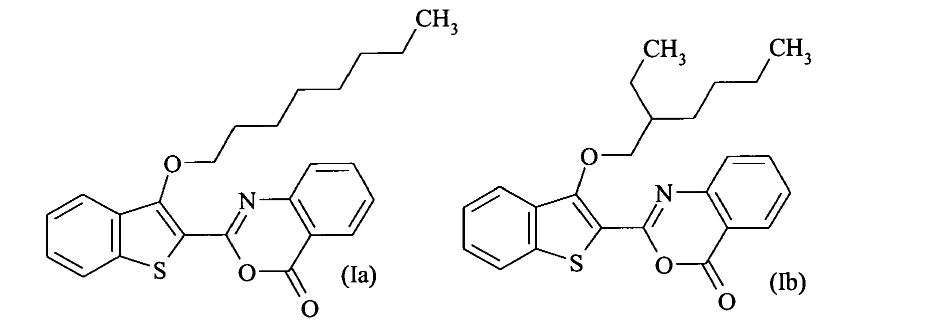

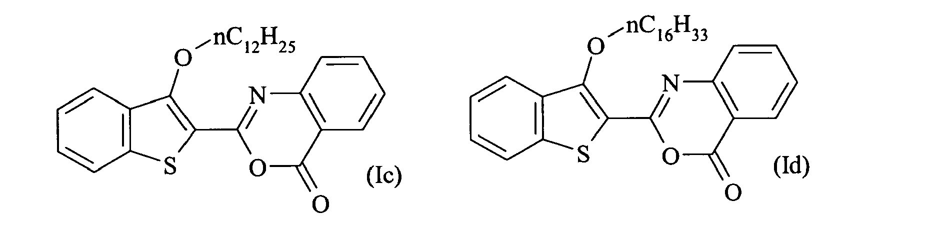

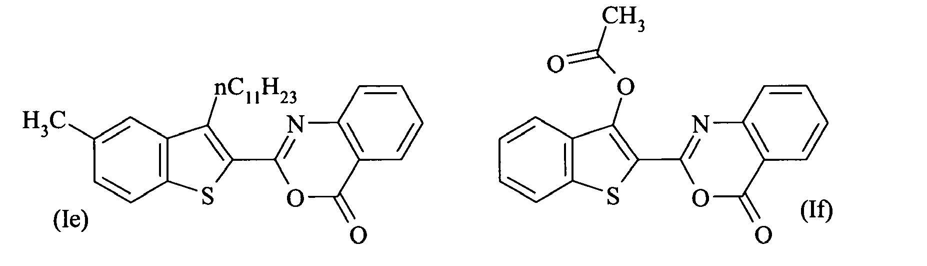

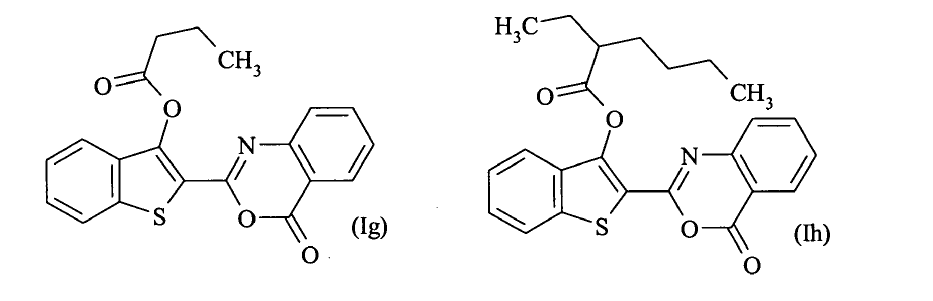

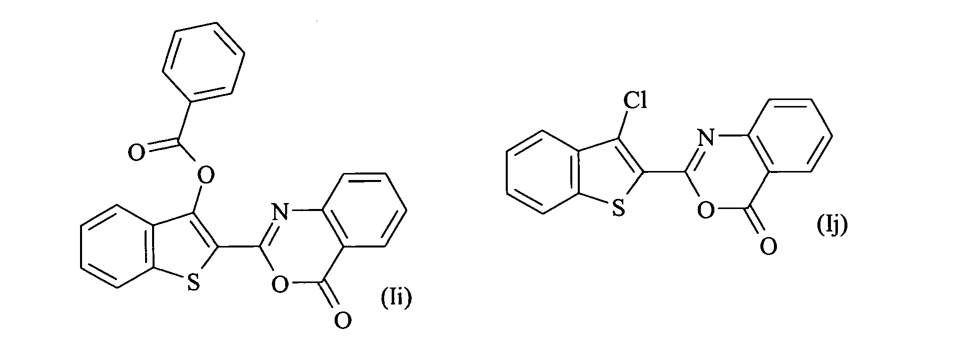

### Preparation of the compounds of formula (I)

The compounds of formula (I) can be prepared following two general methods a) and b). Method a) is a general method compatible with all kind of substituents and the method b) is specifically used when R1 is a carboxylate.

### Method a)

A compound of formula (II) is reacted with a compound of formula (III). The resulting compound of formula (IV) is cyclized to the compound of formula (I).

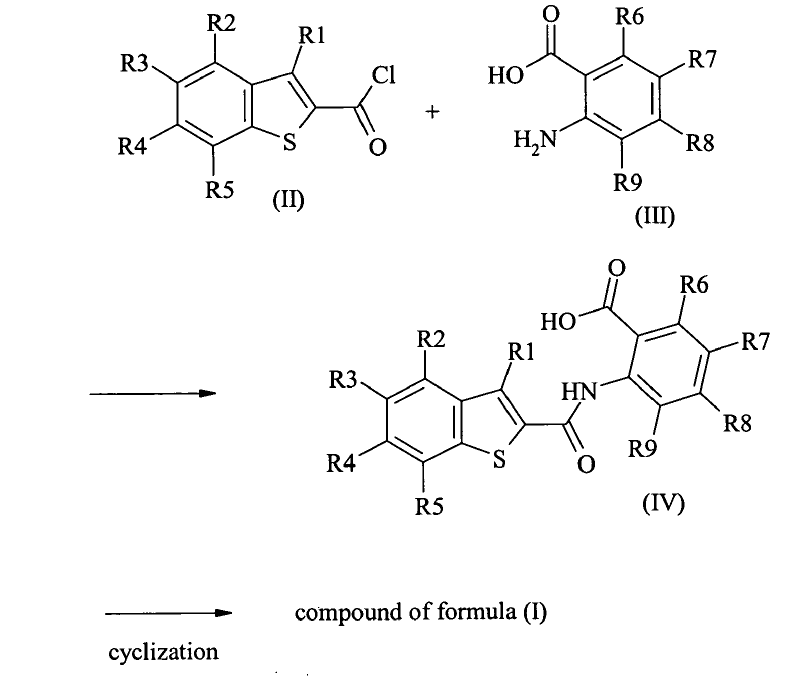

The cyclization is done preferably with acetic anhydride. The solvent is preferably toluene, chlorobenzene, xylene or acetic anhydride, more preferably toluene or acetic anhydride, even more preferably the cyclization is done in acetic anhydride. The cyclization is done preferably at a temperature of from 80 to 160°C, more preferably of from 100 to 145°C, even more preferably under normal pressure and under reflux. The time for cyclization at the desired temperature is preferably of from 30 min to 12 hours, more preferably of from 1 hour to 8 hours.

The compound of formula (II) is prepared by the reaction of thionyl chloride with a compound of formula (V).

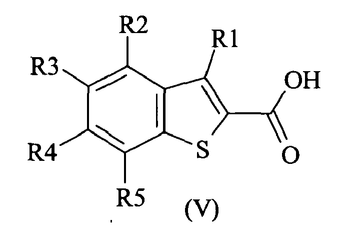

For instance, when R1 is an ether residue, the compound of formula (V) is prepared from a compound of formula (VI) by O-alkylation using the appropriate bromo- or chloroalkane R-X in the presence of a base in a polar solvent. For example, methyl 3-octyl-2- benzothiophenecarboxylate is made by heating methyl 3-hydroxy-2-benzothiophenecarboxylate with 1-bromoctane in a presence of potassium carbonate in N,N-dimethylformamide (DMF). The resulting ester is submitted to saponification with sodium hydroxide or potassium hydroxide in ethanol or in water in the presence of a phase transfer catalyst. The compound of formula (V) is recovered after acidification with a mineral acid such as hydrochloric or sulfuric acid.

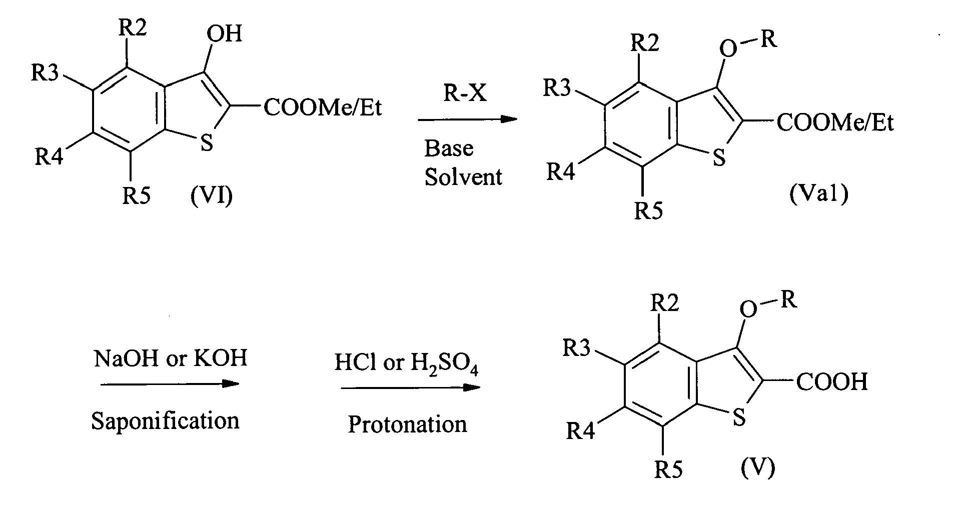

Alkyl 3-hydroxy-2-benzothiophenecarboxylates are known products (for example J.R. Beck, J. Org. Chem. 38, 4086 (1973)).

When R1 is a C1 atom, the corresponding compound of formula (V) with R1 being C1 can be made from the corresponding cinnamic acid by reaction of thionyl chloride in the presence of pyridine (for example T. Higa, A.J. Krubsack, J. Org. Chem. 40, 3037 (1975)).

When R1 is an alkyl or aryl group, the corresponding compound of formula (V) with R1 being alkyl or aryl is made from the corresponding compound of formula (VII), a ketone, which is submitted to base-catalysed cyclisation (the compound of formula (VII) can be, for example, made by Friedel-Crafts acylation of an alkyl arylthioacetate).

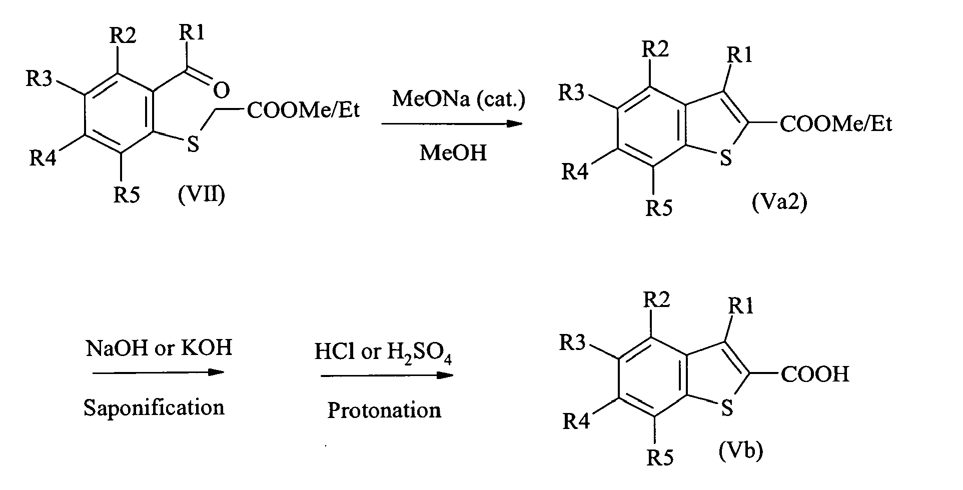

### Method b)

When R1 is OC(O)R12 (a carboxylate), with the R12 being as defined above, also in all its preferred embodiments, the compound of formula (I) is made by reacting a respective compound of formula (X), wherein R2, R3, R4, R5, R6, R7, R8 and R9 are defined as above, also in all their preferred embodiments, with the respective acid anhydride (R12C(O))2O or with the respective acid chloride R12C(O)Cl, with R12 being as defined above, also in all its preferred embodiments, preferably in the presence of an inorganic or organic base, more preferably in the presence of pyridine or sodium acetate.

If the respective acid chloride R12C(O)C1 is used, then the reaction is done preferably in pyridine as solvent; if the respective acid anhydride (R12C(O))20 is used, then the reaction is done preferably in this respective acid anhydride as solvent. The reaction is done preferably at a temperature of from 80 to 160, more preferably of from 100 to 145°C, even more preferably under normal pressure and under reflux. The time for the reaction at the desired temperature is preferably of from 30 min to 12 hours, more preferably of from 1 hour to 8 hours.

The compound of formula (X) is made by reacting a compound of formula (VIII) with a compound of formula (IX) in the presence of a mineral base, preferably potassium carbonate, in polar solvent, preferably in N,N-dimethylformamide (DMF) or N-methylpyrrolidone (NMP), or in water.

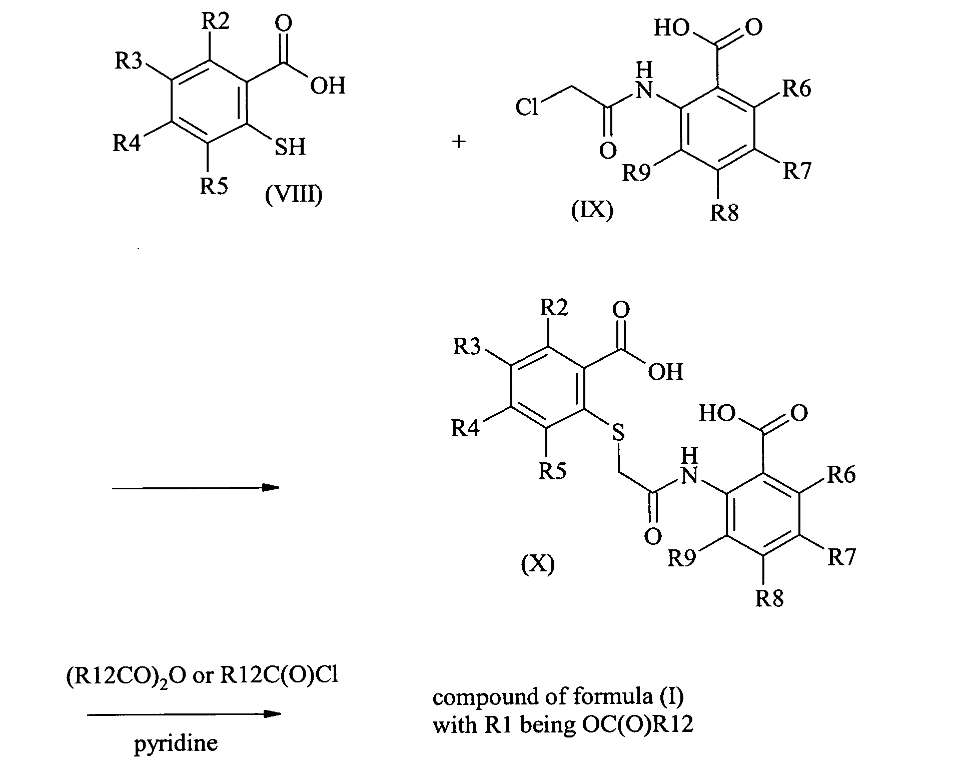

The compounds of formula (I) are used for the stabilization of organic substrates, preferably of plastics, resins, varnishes, coloured compositions such as paints, for example, electrophotographic toners and developers, electret materials, colour filters, and also inks, including printing inks, more preferably plastics and varnishes, preferably for the purpose of stabilizing these high molecular organic polymers against damage or degradation, in particular by oxidation, heat, light or other kinds of radiation, such as electron beams, for example, and in particular by light. Preferred use of the compound of formula (I) is the use as light stabilizers for organic substrates.

One or more compounds of formula (I) can be used simultaneously, preferably one is used.

A further subject of the invention therefore is also a process for the stabilization of organic substrates, also in all their preferred embodiments, characterized by the addition of a compound of formula (I) to the organic substrate. The addition is preferably done by physically mixing the compound of formula (I) with the organic substrate, more preferably the addition is done before or during the manufacture of the organic substrate, or before or during the processing of the organic substrate; even more preferably before or during the extrusion of an organic polymer, or before or during the manufacture of resins, varnishes, paints, electrophotographic toners, developers, electret materials, colour filters and inks.
An case of extrusion, preferably a dry blend is made of the compound of formula (I) with the organic polymer, and then this blend is extruded.
An case of varnishes, paints or inks, preferably the compound of formula (I) is incorporated into the liquid composition before or during it's manufacture, more preferably before or during the dissolution or dispersion of the varnishes, paints or inks.

The compound of formula (I) is preferably added to the organic substrate in an amount of from 0.0001 to 5 % by weight, more preferably of from 0.01 to 1 % by weight, based on the weight of the organic substrate.

Preferred organic substrates are organic polymers, more preferably polar technical polymers, which are known in the literature under the expression "engineering plastics", even more preferably polymers which consist of linear or grafted copolymers.
Examples of organic polymers are:
1. Polymers of monoolefins and diolefins, preferably polypropylene (PP), polyisobutylene, polybut-1-ene, poly-4-methylpent-1-ene, polyisoprene or polybutadiene, as well as polymers of cycloolefins, preferably of cyclopentene or norbornene; furthermore polyethylene (PE) (which optionally can be crosslinked); preferably high density polyethylene (HDPE), polyethylene of high density and high molar weight(HDPE-HMW), polyethylene of high density and ultrahigh molar weight (HDPE-UHMW), medium density polyethylene (HMDPE), low density polyethylene (LDPE), linear low density polyethylene (LLDPE), branched low density polyethylene (BLDPE).
   Polyolefins, i.e. polymers of monoolefins exemplified in the preceding paragraph, in particular polyethylene and polypropylene, can be prepared by various, and especially by the following, methods:
   a) free-radical polymerization (normally under high pressure and at elevated temperature)
   b) catalytic polymerization using a catalyst that normally contains one or more metals of group IVb, Vb, VIb or VIII of the Periodic Table. These metals usually have one or more ligands, such as oxides, halides, alcoholates, esters, ethers, amines, alkyls, alkenyls and/or aryls that may be either π-(pi-) or σ-(sigma-)coordinated. These metal complexes may be in the free form or fixed on substrates, for example on activated magnesium chloride, titanium(III) chloride, alumina or silicon oxide. These catalysts may be soluble or insoluble in the polymerization medium. The catalysts can be active as such in the polymerization or further activators may be used, for example metal alkyls, metal hydrides, metal alkyl halides, metal alkyl oxides or metal alkyloxanes, the metals being elements of groups Ia, IIa and/or IIIa of the Periodic Table. The activators may be modified, for example, with further ester, ether, amine or silyl ether groups. These catalyst systems are usually termed Phillips, Standard Oil Indiana, Ziegler (-Natta), TNZ (DuPont), metallocene or single site catalysts (SSC).
2. Mixtures of the polymers mentioned under point 1., preferably mixtures of polypropylene with polyisobutylene, polyethylene with polyisobutylene, polypropylene with polyethylene (for example PP/HDPE/LDPE) and mixtures of different types of polyethylene (preferably LDPE/HDPE) with one another.
3. Copolymers of monoolefins and diolefins with each other or with other vinyl monomers, preferably ethylene-propylene copolymers, linear low density polyethylene (LLDPE) and mixtures thereof with low density polyethylene (LDPE), propylene-but-1-ene copolymers, propylene-isobutylene copolymers, ethylene-but-1-ene copolymers, ethylene-hexene copolymers, ethylene-methylpentene copolymers, ethylene-heptene copolymers, ethylene-octene copolymers, propylene-butadiene copolymers, isobutylene-isoprene copolymers, ethylenealkyl acrylate copolymers, ethylenealkyl methacrylate copolymers, ethylene-vinyl acetate copolymers (EVA) and their copolymers with carbon monoxide or ethylene-acrylic acid copolymers and their salts (ionomers) as well as terpolymers of ethylene with propylene and a diene such as hexadiene, dicyclopentadiene or ethylidene-norbornene; and mixtures of such copolymers with one another and with polymers mentioned under 1), preferably polypropylene-ethylene-propylene copolymers, LDPE-ethylene-vinyl acetate copolymers, LDPE-ethylene-acrylic acid copolymers, LLDPE-ethylene-vinyl acetate copolymers, LLDPE-ethylene-acrylic acid copolymers and alternating or random polyalkylene-carbon monoxide copolymers and mixtures thereof with other polymers, preferably polyamides.
4. Polymers derived from unsaturated alcohols and amines or the acyl derivatives or acetals thereof, such as polyvinyl alcohol, polyvinyl acetate, polyvinyl stearate, polyvinyl benzoate, polyvinyl maleate, polyvinyl butyral, polyallyl phthalate or polyallyl melamine; as well as their copolymers with olefins mentioned in point 1.
5. Polyacetals such as polyoxymethylene (POM) and those polyoxymethylenes which contain comonomers, preferably ethylene oxide; polyacetals modified with thermoplastic polyurethanes (thermoplastic PUR), acrylates methyl-methacrylate-butadiene-styrene (MBS).
6. Polyphenylene oxides and sulfides, and mixtures thereof with styrene polymers or polyamides.
7. Polyamides (PA) and copolyamides derived from diamines and dicarboxylic acids and/or from aminocarboxylic acids or the corresponding lactams, such as polyamide 4, 6, 6/6, 6/10, 6/9*,* 6/12*,* 4/6, 12/12, 11 and 12, aromatic polyamides starting from m-xylene, diamine and adipic acid; polyamides prepared from hexamethylenediamine and isophthalic and/or terephthalic acid and with or without an elastomer as modifier, preferably poly-2,4,4-trimethylhexamethylene terephthalamide or poly-m-phenylene isophthalamide. Block copolymers of the aforementioned polyamides with polyolefins, olefin copolymers, ionomers or chemically bonded or grafted elastomers; or with polyethers, e.g. with polyethylene glycol, polypropylene glycol or polytetramethylene glycol. As well as polyamides or copolyamides modified with ethylene-propylene-terpolymer (EPDM) or acrylonitrile-butadiene-styrene-polymer (ABS); and polyamides condensed during processing (RIM polyamide systems).
8. Polyureas, polyimides, polyamide-imides, polyether imides, polyester amides, polyhydantoins and polybenzimidazoles.
9. Polyesters derived from dicarboxylic acids and dialcohols and/or from hydroxycarboxylic acids or the corresponding lactones, such as polyethylene terephthalate (PET), polybutylene terephthalate (PBT), poly-1,4-dimethylolcyclohexane terephthalate, polyhydroxybenzoates, as well as block polyether esters derived from hydroxyl-terminated polyethers; and also polyesters modified with polycarbonates or MBS.
10. Polycarbonates (PC) and polyester carbonates.
11. Polysulfones, polyether sulfones and polyether ketones.
12. Crosslinked polymers derived from aldehydes on the one hand and phenols, urea or melamine on the other hand, such as phenol/formaldehyde resins, urea/formaldehyde resins and melamine/formaldehyde resins.
13. Drying and non-drying alkyd resins.
14. Unsaturated polyester resins derived from copolyesters of saturated and unsaturated dicarboxylic acids with polyhydric alcohols and vinyl compounds as crosslinking agents, and also halogen-containing modifications thereof of low flammability.
15. Mixtures (polyblends) of the aforementioned polymers, preferably PP/EPDM, polyamide/EPDM or ABS, polyvinylchloride (PVC)/EVA, PVC/ABS, PVC/MBS, PC/ABS, PBT/ABS, PC/acrylonitile-styrene-acrylester (ASA), PC/PBT, PVC/chlorinated polyethylene (CPE), PVC/acrylates, POM/thermoplastic PUR, PC/thermoplastic PUR, POM/acrylate, POM/MBS, PPO/MBS, polyphenyloxide (PPO)/highimpact polystyrene (HIPS), PPO/PA 6.6 and copolymers, PA/HDPE, PA/PP, PA/PPO, PBT/ABS or PBT/PET/PC.
16. Natural and synthetic organic substances which constitute pure monomeric compounds or mixtures thereof, examples being mineral oils, animal or vegetable fats, oils and waxes, or oils, waxes and fats based on synthetic esters (e.g. phthalates, adipates, phosphates or trimellitates), and also blends of synthetic esters with mineral oils in any desired proportion by weight, as are employed, for example, as spin finishes, and aqueous emulsions thereof.

Preferred examples of organic polymers are:
a) ethylene-vinyl acetate copolymers and their copolymers with carbon monoxide or ethylene-acrylic acid copolymers and their salts (ionomers) as well as terpolymers of ethylene with propylene and a diene such as hexadiene, LDPE-ethylene-vinyl acetate copolymers, LDPE-ethylene-acrylic acid copolymers, LLDPE-ethylene-vinyl acetate copolymers, LLDPE-ethylene-acrylic acid copolymers and alternating or random polyalkylene-carbon monoxide copolymers and mixtures thereof with other polymers, preferably polyamides.
b) Polymers derived from unsaturated alcohols and amines or the acyl derivatives or acetals thereof, such as polyvinyl alcohol, polyvinyl acetate, polyvinyl stearate, polyvinyl benzoate, polyvinyl maleate, polyvinyl butyral, polyallyl phthalate or polyallyl melamine; as well as their copolymers with olefins.
c) Polyacetals such as polyoxymethylene and those polyoxymethylenes which contain comonomers, preferably ethylene oxide; polyacetals modified with thermoplastic polyurethanes.
d) Polyphenylene oxides and sulfides, and mixtures thereof with styrene polymers or polyamides.
e) Polyamides and copolyamides derived from diamines and dicarboxylic acids and/or from aminocarboxylic acids or the corresponding lactams, such as polyamide 4, 6, 6/6, 6/10, 6/9, 6/12, 4/6, 12/12, 11 and 12, aromatic polyamides starting from m-xylene, diamine and adipic acid; polyamides prepared from hexamethylenediamine and isophthalic and/or terephthalic acid and with or without an elastomer as modifier, preferably poly-2,4,4-trimethylhexamethylene terephthalamide or poly-m-phenylene isophthalamide. Block copolymers of the aforementioned polyamides with polyolefins, olefin copolymers, ionomers or chemically bonded or grafted elastomers; or with polyethers, e.g. with polyethylene glycol, polypropylene glycol or polytetramethylene glycol. As well as polyamides or copolyamides.
f) Polyureas, polyimides, polyamide-imides, polyether imides, polyester amides, polyhydantoins and polybenzimidazoles.
g) Polyesters derived from dicarboxylic acids and dialcohols and/or from hydroxycarboxylic acids or the corresponding lactones, such as polyethylene terephthalate, polybutylene terephthalate, poly-1,4-dimethylolcyclohexane terephthalate, polyhydroxybenzoates, as well as block polyether esters derived from hydroxyl-terminated polyethers; and also polyesters modified with polycarbonates.
h) Polycarbonates and polyester carbonates.
i) Polysulfones, polyether sulfones and polyether ketones.
j) Crosslinked polymers derived from aldehydes on the one hand and phenols, urea or melamine on the other hand, such as phenol/formaldehyde resins, urea/formaldehyde resins and melamine/formaldehyde resins.
k) Drying and non-drying alkyd resins.
l) Unsaturated polyester resins derived from copolyesters of saturated and unsaturated dicarboxylic acids with polyhydric alcohols and vinyl compounds as crosslinking agents, and also halogen-containing modifications thereof of low flammability.
m) Mixtures (polyblends) of the aforementioned polymers, preferably PP/EPDM, polyamide/EPDM, PVC/EVA, PC/PBT, PVC/CPE, PC/thermoplastic PUR, PA/HDPE, PA/PP, PA/PPO, PBT/PET/PC.
n) Natural and synthetic organic substances which constitute pure monomeric compounds or mixtures thereof, examples being mineral oils, animal or vegetable fats, oils and waxes, or oils, waxes and fats based on synthetic esters (e.g. phthalates, adipates, phosphates or trimellitates), and also blends of synthetic esters with mineral oils in any desired proportion by weight, as are employed, for example, as spin finishes, and aqueous emulsions thereof.

Especially preferred organic polymers are ethylene-vinylacetat coplymers (EVA), polycarbonates or polyesters;
in particular a EVA;
further in particular a polyethyleneterephthalate (PET);
and further in particular a polybutyleneterephthalate (PBT).

Primarily the compounds of formula (I) according to the invention are suitable for organic polymers selected from the group of so called engineering plastics.

The compounds of formula (I) possess the required characteristics as efficient UV absorbers. They are soluble in common organic solvents. The common structural feature allows a broad variation of the polarity of the molecules depending on the substitution pattern. This fact enables this class of compounds to be used broadly for applications in a large variety of polymers including their use for coating applications which is an improvement over commercially available UV absorbers. The compounds of formula (I) are characterized by
- melting points in the range of from 60 to 220°C,
- absorption maxima typically in the UV-A range between 300 nm and 400 nm,
- high molar extinction coefficients at their absorption maxima in the range of from 15000 to at about 40000 l/mol*cm,
- decomposition temperatures above 280°C and in many cases up to 380°C and even higher.

The new compounds according to the invention provide excellent stabilization against damage by light or oxidation and therefore are able to protect organic substrates against deterioration by degrading environmental influences.

The use of the compounds of formula (I) allows a long term maintenance of properties of the organic substrates in which they are used, including appearance and in particular the maintenance of color, gloss and transparency, which are highly important parameters of organic polymers, especially of technical polymers.

### Examples

### UV/vis

λₘₐₓ (lambda max) and ε (epsilon) values of the compounds of formula (I) are determined dissolved in CH₂Cl₂ using an UV spectrophotometer. These values are obtained by balancing the measurements performed on compound solutions at three different concentrations.

### Melting point (MP)

The melting point (MP) is determined using a Büchi B-545 Melting Point apparatus.

### Decomposition point (DP)

The decomposition point (DP) is determined using a TA Instruments DSC Q100 apparatus, the compound being incorporated into a sealed aluminum pan. Analysis conditions are as following: Temperature range from 40 to 400°C, heating rate 10°C/min, nitrogen flow of 50 ml/min. Values are determined by single measurement.

### Used commercial products:

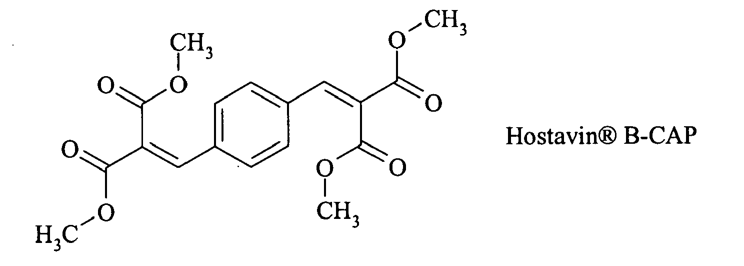

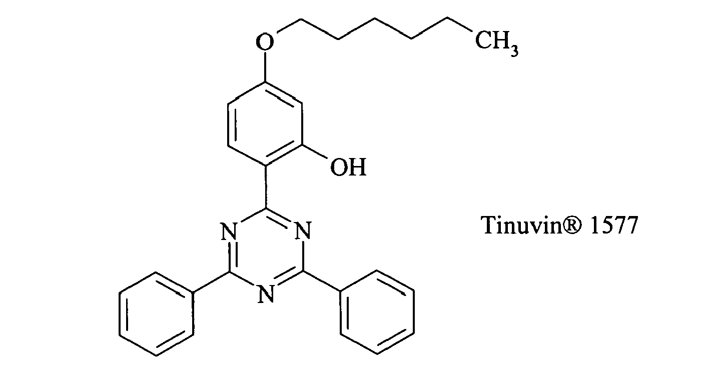

Aliquat® 336 (Henkel Corp.) is methyltrialkyl (C₈-C₁₀)ammonium chloride, average MW = 442 g/mol

### Example 1

### O-alkylation and hydrolysis

A mixture of 20.8 g of methyl 3-hydroxy-2-benzothiophenecarboxylate, 21.44 g of 1-bromooctane and 20.7 g potassium carbonate in 100 ml of DMF was stirred at 120°C for 1h. The mixture was poured in 11 of water and the product was extracted three times with each 200 ml of cyclohexane. The combined organic layers were washed with 1 M aqueous hydrochloric acid solution and then with water, and were dried with MgSO₄ and then evaporated in vacuo. 31 g of methyl 3-octyloxy-2-benzothiophenecarboxylate were obtained as an oily liquid.
To this liquid is added 50 ml of 30% aqueous sodium hydroxide solution, 50 ml of water and 0.5 g of Aliquat® 336.
The mixture was vigorously stirred for 45 minutes at 100°C. After cooling,
the mixture was acidified with concentrated aqueous hydrochloric acid and the formed precipitate was filtered and washed with water and dried at 80°C in vacuo. 25 g of 3-octyloxy-2-benzothiophenecarboxylic acid were obtained as a white solid.

### Acid chloride formation and subsequent reaction with anthranilic acid.

23.3 g of 3-octyloxy-2-benzothiophenecarboxylic acid were reacted with 59 ml of thionyl chloride in the presence of 5 drops of DMF at 75°C for 45 minutes. Excess thionyl chloride was removed by distillation.
The crude acid chloride was then dissolved in 40 ml of toluene. The obtained solution was then poured into a mixture of 9.9 g of anthranilic acid, 8 ml of 30% by weight aqueous sodium hydroxide solution, 80 ml of water and 0.5 g of benzyltributylammonium chloride in the course of 30 minutes. The mixture was then extracted three times with each 70 ml of dichloromethane. The combined organic layers were washed with 1 M aqueous hydrochloric acid solution, then with water, and were dried by means of MgSO₄ and then evaporated in vacuo. 31.3 g of 2-(3-octyloxy-2-benzothiophenecarbonamido)-benzoic acid were obtained as an oily liquid.

### Cyclisation

A mixture of 31.3 g of 2-(3-octyloxy-2-benzothiophenecarbonamido)-benzoic acid, 89.6 g of acetic anhydride and 270 ml of toluene was stirred at 108°C for 6 h. After cooling, the mixture was washed two times with each 100 ml of water, then two times with each 50 ml of a 4% by weight aqueous sodium hydrogenocarbonate solution, then dried with MgSO₄ and then evaporated in vacuo. The resulting crude was then recrystallised in ethanol. 15 g of the compound of formula (Ia) were obtained as a white powder.
MP = 89°C, DP = 370°C, λₘₐₓ = 341 nm, εₘₐₓ = 26000 l/mol*cm.
Solubility: in xylene > 100 g/l;
in Dowanol^{™} PMA (CH₃COO₂CH(CH₃)CH₂OCH₃) > 50 g/l.

### Example 2

### O-alkylation and hydrolysis

3-(2-ethylhexyloxy)-2-benzothiophenecarboxylic acid was prepared according to the procedure of example 1 by replacing 1-bromooctane by 1-bromo-2-ethylhexane.
14.5 g of 3-(2-ethylhexyloxy)-2-benzothiophenecarboxylic acid were obtained.

### Acid chloride formation and subsequent reaction with anthranilic acid.

2-(3-(2-ethylhexyloxy)-2-benzothiophenecarbonamido)-benzoic acid was prepared according to the procedure of example 1 by replacing 2-(3-octyloxy-2-benzothiophenecarbonamido)-benzoic acid by 2-(2-ethylhexyloxy)-2-benzothiophenecarbonamido)-benzoic acid.
30.8 g of 2-(3-(2-ethylhexyloxy)-2-benzothiophenecarbonamido)-benzoic acid were obtained.

### Cyclisation

The compound of formula (Ib) was prepared according to the procedure of example 1 by replacing 2-(3-octyloxy-2-benzothiophenecarbonamido)-benzoic acid by 2-(3-(2-ethylhexyloxy)-2-benzothiophenecarbonamido)-benzoic acid.
14.8 g of the compound of formula (Ib) were obtained.
MP = 69°C, DP 385°C, λₘₐₓ = 341 nm, εₘₐₓ = 26200 l/mol*cm.
Solubility: in xylene > 100 g/1;
in Dowanol^{™} PMA (CH₃COO₂CH(CH₃)CH₂OCH₃) > 50 g/l.

### Example 3

### O-alkylation and hydrolysis

3-dodecyloxy-2-benzothiophenecarboxylic acid was prepared according to the procedure of example 1 by replacing 1-bromooctane by 1-bromododecane.
34.8 g of 3-dodecyloxy-2-benzothiophenecarboxylic acid were obtained.

### Acid chloride formation and subsequent reaction with anthranilic acid.

2-(3-dodecyloxy-2-benzothiophenecarbonamido)-benzoic acid was prepared according to the procedure of example 1 by replacing 3-octyloxy-2-benzothiophenecarboxylic acid by 3-dodecyloxy-2-benzothiophenecarboxylic acid.
26.5 g of 2-(3-dodecyloxy-2-benzothiophenecarbonamido)-benzoic acid were obtained.

### Cyclisation

The compound of formula (Ic) was prepared according to the procedure of example 1 by replacing 2-(3-octyloxy-2-benzothiophenecarbonamido)-benzoic acid by 2-(3-dodecyloxy-2-benzothiophenecarbonamido)-benzoic acid.
23.2 g of the compound of formula (Ic) were obtained.
MP = 81°C, DP 381°C, λₘₐₓ = 342 nm, εₘₐₓ = 21800 l/mol*cm.
Solubility: in xylene : 120 g/l;

### Example 4:

### O-alkylation and hydrolysis

3-hexadecyloxy-2-benzothiophenecarboxylic acid was prepared according to the procedure of example 1 by replacing 1-bromooctane by 1-bromohexadecane.
40.5 g of 3-hexadecyloxy-2-benzothiophenecarboxylic acid were obtained.

### Acid chloride formation and subsequent reaction with anthranilic acid.

2-(3-hexadecyloxy-2-benzothiophenecarbonamido)-benzoic acid was prepared according to the procedure of example 1 by replacing 3-octyloxy-2-benzothiophenecarboxylic acid by 3-hexadecyloxy-2-benzothiophenecarboxylic acid. 38 g of 2-(3-hexadecyloxy-2-benzothiophenecarbonamido)-benzoic acid were obtained.

### Cyclisation

The compound of formula (Id) was prepared according to the procedure of example 1 by replacing 2-(3-octyloxy-2-benzothiophenecarbonamido)-benzoic acid by 2-(3-hexadecyloxy-2-benzothiophenecarbonamido)-benzoic acid.
23.8 g of the compound of formula (Id) were obtained.
MP = 83°C, DP 384°C, λₘₐₓ = 341 nm, εₘₐₓ = 31900 l/mol*cm.
Solubility: in xylene : 100 g/l;

### Examples 5

### Electrophilic acylation

Into a suspension of 40 g aluminium chloride in 40 ml 1,2-dichloroethane was dropped 58 ml of lauroyl chloride at 0°C. The resulting suspension was then stirred at room temperature for 1 h and cooled down to 0°C. A solution of 25.2 g of ethyl 4-tolylthioglycolate in 70 ml of 1,2-dichloroethane was dropwise added in the course of 1 h. The mixture was then stirred at 45°C for 3h and was poured onto a mixture of 300 ml of water with 80 ml of concentrated aqueous hydrochloric acid. The mixture was extracted with dichloromethane, the organic layer was washed two times with each 100 ml of water and once with 100 ml of a saturated aqueous solution of sodium hydrogenocarbonate, then it was dried with MgSO₄ and evaporated in vacuo.
70 g of ethyl (4-methyl-2-dodecanoylphenyl)-thioglycolate as a yellow liquid were obtained.

### Benzothiophene formation and hydrolysis

Into a suspension of 70 g of ethyl (4-methyl-2-dodecanoylphenyl)-thioglycolate in 75 ml of methanol was dropped 14 ml of a 30% by weight methanolic sodium methylate solution. The mixture was refluxed for 1h, cooled to room temperature and neutralized with 6 ml of acetic acid. The suspension was then filtered, washed with methanol and dried in vacuo. 34 g of ethyl 5-methyl-3-undecyl-2-benzothiophenecarboxylate as a white solid were obtained.
This carboxylate was dissolved in a solution of 6.4 g of potassium hydroxide in 100 ml of ethanol. The solution was refluxed for 1h, then acidified with aqueous hydrochloric acid. The ethanol was evaporated in vacuo and the resulting suspension was filtered, washed with water and dried at 45°C in vacuo.
27.5 g of 5-methyl-3-undecyl-2-benzothiophenecarboxylic acid were obtained.

### Acid chloride formation and subsequent reaction with anthranilic acid.

2-(5-methyl-3-undecyl-2-benzothiophenecarbonamido)-benzoic acid was prepared according to the procedure of example 1, replacing 3-octyloxy-2-benzothiophenecarboxylic acid by 5-methyl-3-undecyl-2-benzothiophenecarboxylic acid.
22.7 g of 2-(5-methyl-3-undecyl-2-benzothiophenecarbonamido)-benzoic acid were obtained.

### Cyclisation

The compound of formula (Ie) was prepared according to the procedure of example 1 by replacing 2-(3-octyloxy-2-benzothiophenecarbonamido)-benzoic acid by 2-(5-methyl-3-undecyl-2-benzothiophenecarbonamido)-benzoic acid.
19.4 g of the compound of formula (Ie) were obtained.
MP = 117°C, no decomposition below 400°C, λₘₐₓ = 343 nm, εₘₐₓ = 25900 l/mol*cm.
Solubility: in xylene = 40 g/1.

### Example 6

### Nucleophilic substitution

A mixture of 2-(2-chloroacetamido)-benzoic acid (21.4 g), thiosalicylic acid (15.4 g) and potassium carbonate (41.1 g) in DMF (350 ml) was stirred at 130°C for 1 h. After cooling, the mixture was poured into 4000 ml of water and this solution was acidified with aqueous hydrochloric acid. The resulting suspension was filtered and the solid was washed with water and dried at 70°C in vacuo.
29.6 g of compound of formula (1) as a white solid were obtained.

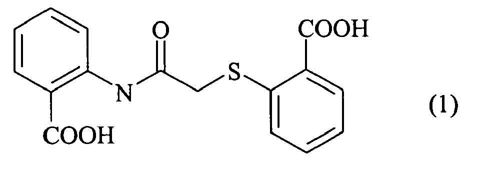

### Condensation reaction

A mixture of 16.6 g of compound of formula (1), 12.3 g sodium acetate and 75 ml acetic anhydride was stirred at 133°C for 3h. The hot mixture was then poured into 300 ml water. The subsequent suspension was filtered and the solid washed with water and dried in vacuo at 100°C.
15.1 g of compound of formula (If) as a white solid were obtained.
MP = 210°C, DP 351°C, λₘₐₓ = 337 nm, εₘₐₓ = 28700 l/mol*cm.
Solubility: in xylene < 10 g/l.

### Example 7

### Condensation reaction

A mixture of 16.56 g of compound of formula (1) and 123 ml butyric anhydride was stirred at 140°C for 2h. The hot mixture was then poured into 400 ml water. The resulting suspension was filtered and the solid washed with water and dried at 100°C in vacuo.
7.6 g of compound of formula (Ig) as a white solid were obtained.
MP = 177°C, DP 352°C, λₘₐₓ = 337 nm, εₘₐₓ = 26400 l/mol*cm.
Solubility: in xylene < 10 g/l.

### Example 8

### Condensation reaction

To a solution of 14.63 g of compound of formula (1) in 75 ml pyridine was added 38.5 ml 2-ethylhexanoyl chloride. The mixture was stirred at 120°C for 5 h. The hot mixture was then poured into 400 ml water. The subsequent suspension was filtered and the solid washed with water and dried at 100°C in vacuo.
13.8 g of the compound of formula (Ih) as a white solid were obtained.
MP = 143°C, DP 375°C, λₘₐₓ = 337 nm, εₘₐₓ = 24100 l/mol*cm.
Solubility: in xylene = 10 g/1.

### Example 9

### Condensation reaction

To a solution of 16.56 g of compound of formula (1) in 75 ml pyridine was added 29 ml of benzoyl chloride, and the mixture was stirred at 120°C for 5 h. The hot mixture was then poured into 400 ml water. The subsequent suspension was filtered and the solid washed with water and dried at 100°C in vacuo.
18.9 g of compound of formula (Ii) as a white solid were obtained.

MP = 220°C, no decomposition below 400°C, λₘₐₓ = 338 nm, εₘₐₓ = 25500 l/mol*cm. Solubility: in xylene < 10 g/l.

### Examples 10

### Condensation reaction

### Reaction with anthranilic acid

2-(3-chloro-2-benzothiophenecarbonamido)-benzoic acid was prepared according to the procedure of example 1 (amide formation) using 3-chloro-2-benzothiophenecarbonyl chloride as acid chloride.
31.8 g of 2-(3-chloro-2-benzothiophenecarbonamido)-benzoic acid were obtained.

### Cyclisation

The compound of formula (Ij) was prepared according to the procedure of example 1 by replacing 2-(3-octyloxy-2-benzothiophenecarbonamido)-benzoic acid by 2-(3-chloro-2-benzothiophenecarbonamido)-benzoic acid.
24.7 g of the compound of formula (Ij) were obtained.
MP = 213°C, no decomposition point below 390°C, λₘₐₓ = 338 nm, εₘₐₓ = 29000 l/mol*cm. Solubility in xylene < 5 g/l

### Application Examples in Polycarbonate

### Test Conditions

Artificial weathering was carried out according to DIN 53387 in a Weather-O-Meter (producer Atlas) equipped with Xe lamps and internal as well as external filters based on borosilicate. In such instrument a light intensity of 0.47 W/m² at a wavelength of 340 nm is reached. The duration of an exposure cycle is defined to be 120 minutes including a dry period of 102 min at 50% relative humidity and a black panel temperature of 63°C, whereas the duration of the water spray on period is defined being 18 min at 95% relative humidity and a black panel temperature of 58°C.

Treatments by aging under artificial UV-irradiation have been carried out in order to study the influence of the various UV absorbers and UV absorber-combinations. For this purpose UV-CON A treatment according to ASTM D 5208, cycle A has been used containing fluorescent lamps with light emission λ < 340nm. The cycles are characterized by exposure for 20 hours at 50°C without water condensation followed by a period of 4 hours at 40°C with water condensation.

Aging at elevated temperatures (oven aging) was carried out in an air draft oven according to DIN 53 383 at 120°C, if not otherwise stated.

An important physical and technical parameter is the transparency, which has to be maintained during a long service time at a level as high as possible. The samples have been investigated before and after artificial UV-A-exposure. The transparency measurements took place according to "Standard Test Methods for Transparency of Plastic Sheeting", ASTM designation D 1746-96; current edition approved Aug. 10, 1996, published February 1997; originally published as D 1746-60. For the measurements a spectrophotometer, type Minolta CM 3500 D, has been used at wavelength of 700 nm. The difference between the unexposed plaques and the exposed plaques is determined. High values correspond to high transparency.

Among other tests the physical evaluation of the artificially aged samples took place by gloss measurements which have been executed according to the rules of the "Standard Test Method for Specular Gloss of Plastic Films and Solid Plastics", ASTM designation D 2457-97. This method is under designation of ASTM Committee D-20 on Plastics and is the direct responsibility of Subcommittee D20.40 on Optical Properties. Current edition approved Jan. 10, 1997, published May 1997. The method describes procedures for the measurements of gloss of plastic films and solid plastics, both opaque and transparent, containing separate gloss angles. The instrument used was a gloss-meter type micro-TRI-gloss, producer Byk-Gardener. The difference between the unexposed plaques and the exposed plaques is determined. High values correspond to high gloss.

Tensil strength was measured according to DIN 53 455. The higher the value the better.

Elongation at break was measured according to DIN 53 571. The higher the value the better.

The amounts in % of the UV-absorbers in the examples are given in % by weight, based on the weight of the total composition (UV-absorbers with polymer), if not otherwise stated.

### Application Example 1: Effects on injection molded Polycarbonate

100 parts of polycarbonate, type Lexan® 141 (producer General Electric) have been dry blended with the UV-absorbers, details are shown in tables 1 to 4. Afterwards the individual mixtures have been pre-extruded in a single screw extruder (screw composition 1:3) applying 4 temperature zones (ramping mode) heated from 260 °C to 280°C with a die width of 4 mm and a speed of rotation of 80 rpm. These pre-extruded formulations have been finally used to prepare plaques (dimensions 100 x 100 x 1mm) by means of injection molding (IM machine type Arburg) at 280°C with a pressure of 50 bars.

Tables 1 and 2 show results after artificial weathering. The compound of formula (Ic) gives better results with regard to gloss and the compound of formula (Id) with regard to transparency, compared with the virgin polymer or with the two commercial UV absorbers.

| Table 1 Artificial Weathering (DIN 53387) of Polycarbonate plaques Impact on gloss (60°) | | | | |
|---|---|---|---|---|
| Exposure time in hours | virgin polymer | Hostavin^{®}B-CAP 0.2 % | Tinuvin^{®}1577 0.2 % | Compound of formula (Ic) 0.2 % |
| 0 | 165 | 170 | 172 | 173 |
| 50 | 162 | 167 | 171 | 174 |
| 100 | 163 | 169 | 172 | 175 |

| Table 2 Artificial Weathering (DIN 53387) of Polycarbonate plaques Impact on transparency (%) | | | | |
|---|---|---|---|---|
| Exposure time in hours | virgin polymer | Hostavin^{®}B-CAP 0.2 % | Tinuvin^{®}1577 0.2 % | Compound of formula (Id) 0.2% |
| 0 | 90.14 | 90.09 | 90.11 | 91.18 |
| 50 | 90.11 | 90.16 | 90.08 | 91.16 |
| 100 | 90.04 | 90.14 | 90.00 | 91.11 |

Table 3 shows the impact of UV-A exposure on transparency. Again, compared with the virgin polymer as well as with two commercial UV absorbers, the compound of formula (Ib) shows superior performance.

| Table 3 Artificial UV irradiation (UV-CON A) (ASTM D 5208) of Polycarbonate plaques Impact on transparency (%) | | | | |
|---|---|---|---|---|
| Exposure time in hours | virgin polymer | Hostavin^{®}B-CAP 0.2 % | Tinuvin^{®}1577 0.2 % | Compound of formula (Ib) 0.2 % |
| 0 | 90.19 | 90.26 | 90.22 | 90.28 |
| 50 | 90.10 | 90.18 | 90.11 | 90.21 |
| 100 | 90.04 | 90.18 | 90.15 | 90.20 |

Not only under conditions of light exposure but also under heat treatment in the oven, formulations with compound of formula (I) show superior efficiency compared with the virgin polymer and with the two commercial UV absorbers. Results with regard to transparency are in table 4.

| Table 4 Oven aging of Polycarbonate plaques at T= 120°C Impact on transparency (%) | | | | |
|---|---|---|---|---|
| Exposure time in days | virgin polymer | Hostavin^{®} B-CAP 0.2 % | Tinuvin^{®}1577 0.2 % | Compound of formula (Ib) 0.2% |
| 0 | 90.14 | 90.19 | 90.21 | 90.27 |
| 1 | 90.15 | 90.10 | 90.14 | 90.20 |
| 2 | 90.09 | 90.07 | 89.94 | 90.12 |
| 3 | 90.02 | 89.98 | 89.85 | 90.04 |

### Application example 2

100 parts of polycarbonate, type Lexan® 141 (producer General Electric) have been dried for 4 hours at 120°C and than dry blended with the UV-absorbers shown in tables 5 to 9. Afterwards the individual mixtures have been pre-extruded in a single screw extruder (screw composition 1:3) applying 4 temperature zones (ramping mode) heated from 260 °C to 280°C with a die width of 3 mm and a speed of rotation of 50 rpm. These pre-extruded formulations have been finally used to prepare plaques (dimensions 100 x 100 x 1mm) by means of injection molding (IM machine type Arburg) at 280°C with a pressure of 50 bars.

Table 5 shows results after artificial weathering (WOM CAM-7). The compounds of formula (Ia) and (Ib) give better results with regard to tensile strength compared with a sample which contains the benzoxazinone compound YT 35/03 being prepared according to WO 2005/118562.

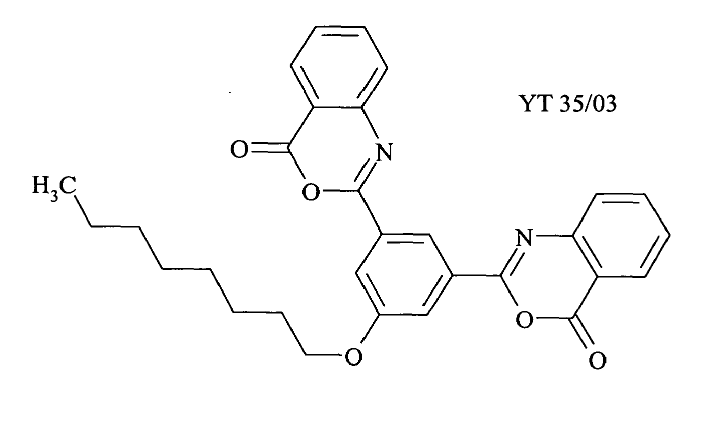

| Table 5 Artificial Weathering (DIN 53387) of Polycarbonate plaques Impact on tensile strength (MPa) | | | |
|---|---|---|---|
| Exposure time in hours | YT 35/03 0.2 % | Compound of formula (Ia) 0.2 % | Compound of formula (Ib) 0.2 % |
| 0 | 62.6 | 69.5 | 66.7 |
| 50 | 66.1 | 70.2 | 71.7 |
| 100 | 64.1 | 74.7 | 72.4 |

Table 6 shows results after long term UV-A exposure (UV-CON A; ASTM D 5208). The compound of formula (Ia) gives better results with regard to gloss (60°) compared with a sample which contains the benzoxazinone compound YT 35/03.

| Table 6 Artificial UV irradiation (UV-CON A; ASTM D 5208) of Polycarbonate plaques - Impact on gloss (60°) | | |
|---|---|---|
| Exposure time in hours | YT 35/03 0.2 % | Compound of formula (Ia) 0.2% |
| 0 | 166 | 172 |
| 50 | 163 | 167 |
| 100 | 161 | 168 |
| 150 | 163 | 172 |
| 200 | 161 | 168 |

Table 7 shows results after long term heat exposure (oven aging) at 120°C. The compound of formula (Ib) gives better results with regard to gloss (60°) compared with a sample which contains the benzoxazinone compound YT 35/03.

| Table 7 Oven aging of Polycarbonate plaques at T= 120°C Impact on transparency (%) | | |
|---|---|---|
| Exposure time in days | YT 35/03 0.2 % | Compound of formula (Ib) 0.2% |
| 0 | 163 | 171 |
| 1 | 169 | 176 |
| 2 | 168 | 181 |
| 3 | 175 | 177 |
| 4 | 166 | 174 |
| 5 | 169 | 176 |

Table 8 shows results after long term UV-A exposure (UV-CON A; ASTM D 5208). The compound of formula (Ia) gives better results with regard to tensile strength compared with a sample which contains the benzoxazinone compound YT 35/03.

| Table 8 Artificial UV irradiation (UV-CON A; ASTM D 5208) of Polycarbonate plaques Impact on tensile strength (MPa) | | |
|---|---|---|
| Exposure time in hours | YT 35/03 0.2 % | Compound of formula (Ia) 0.2% |
| 0 | 62.6 | 69.5 |
| 50 | 66.3 | 69.1 |
| 100 | 69.4 | 70.8 |

Table 9 shows results after long term UV-A exposure (UV-CON A; ASTM D 5208). The compound of formula (Ia) gives better results with regard to elongation at break compared with a sample which contains the benzoxazinone compound YT 35/03.

| Table 9 Artificial UV irradiation (UV-CON A; ASTM D 5208) of Polycarbonate plaques Impact on elongation at break (%) | | |
|---|---|---|
| Exposure time in hours | YT 35/03 0.2 % | Compound of formula (Ia) 0.2% |
| 50 | 68.2 | 73.2 |
| 100 | 51.2 | 54.0 |

## Claims

1. A compound of formula (I), 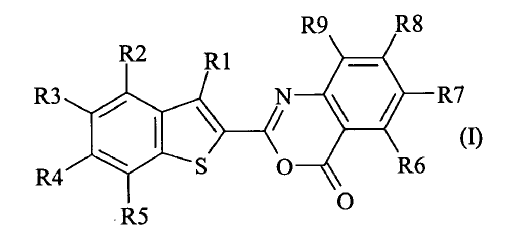 wherein
R1, R2, R3, R4, R5, R6, R7, R8 and R9 are same or different and independently from each other selected from the group consisting of H, CN, halogen, OH,
C₁₋₁₈ alkyl, the C₁₋₁₈ alkyl being unsubstituted or substituted by C₁₋₈ alkyl, C₁₋₈ alkenyl, C₁₋₈ alkoxy, halogen, OH or C₆₋₁₂ aryl,
C₁₋₁₈ alkenyl, C₃₋₁₈ cycloalkyl, C₃₋₁₈ cycloalkenyl,
C₆₋₁₂ aryl, the C₆₋₁₂ aryl being unsubstituted or substituted by C₁₋₈ alkyl, C₁₋₈ alkenyl, C₁₋₈ alkoxy, halogen or OH,
OC₁₋₁₈ alkyl, the C₁₋₁₈ alkyl residue being unsubstituted or substituted by C₁₋₈ alkyl, C₁₋₈ alkenyl, OH, C₆₋₁₂ aryl or NR10R11,
OC₆₋₁₂ aryl, the C₆₋₁₂ aryl residue being unsubstituted or substituted by C₁₋₈ alkyl, C₁₋₈ alkenyl, OH or NR10R11, and
OC(O)R12;
R12 is
C₁₋₁₈ alkyl, the C₁₋₁₈ alkyl being unsubstituted or substituted by C₁₋₈ alkyl, C₁₋₈ alkenyl, OH, C₆₋₁₂ aryl or NR10R11, or R12 is
C₁₋₁₂ aryl, the C₁₋₁₂ aryl residue being unsubstituted or substituted by C₁₋₈ alkyl, C₁₋₈ alkenyl, OH, or NR10R11;
the R10 and R11 residues are same or different and independently from each other selected from the group consisting of H, C₁₋₈ alkyl, C₁₋₈ alkenyl and C₆₋₁₂ aryl.

2. A compound of formula (I) according to claim 1, wherein
R1 is selected from the group consisting of H, CN, halogen, OH,
C₁₋₁₈ alkyl, the C₁₋₁₈ alkyl being unsubstituted or substituted by C₁₋₈ alkyl, C₁₋₈ alkenyl, C₁₋₈ alkoxy, halogen, OH or C₆₋₁₂ aryl,
C₁₋₁₈ alkenyl, C₃₋₁₈ cycloalkyl, C₃₋₁₈ cycloalkenyl,
C₆₋₁₂ aryl, the C₆₋₁₂ aryl being unsubstituted or substituted by C₁₋₈ alkyl, C₁₋₈ alkenyl, C₁₋₈ alkoxy, halogen or OH,
OC₁₋₁₈ alkyl, the C₁₋₁₈ alkyl residue being unsubstituted or substituted by C₁₋₈ alkyl, C₁₋₈ alkenyl, OH, C₆₋₁₂ aryl or NR10R11,
OC₆₋₁₂ aryl, the C₆₋₁₂ aryl residue being unsubstituted or substituted by C₁₋₈ alkyl, C₁₋₈ alkenyl, OH or NR10R11, and
OC(O)R12;
R12 is
C₁₋₁₈ alkyl, the C₁₋₁₈ alkyl being unsubstituted or substituted by C₁₋₈ alkyl, C₁₋₈ alkenyl, OH, C₆₋₁₂ aryl or NR10R11, or R12 is
C₁₋₁₂ aryl, the C₁₋₁₂ aryl residue being unsubstituted or substituted by C₁₋₈ alkyl, C₁₋₈ alkenyl, OH, or NR10R11;
R2, R4, R5, R6, R7, R8 and R9 are H;
R3 is selected from the group consisting of H, CN, halogen, OH,
C₁₋₁₈ alkyl, the C₁₋₁₈ alkyl being unsubstituted or substituted by C₁₋₈ alkyl, C₁₋₈ alkenyl, C₁₋₈ alkoxy, halogen or C₆₋₁₂ aryl, and
OC₁₋₁₈ alkyl, the C₁₋₁₈ alkyl residue being unsubstituted or substituted by C₁₋₈ alkyl or C₆₋₁₂ aryl;
the R10 and R11 residues are same or different and independently from each other selected from the group consisting of H, C₁₋₈ alkyl, C₁₋₈ alkenyl and C₆₋₁₂ aryl.

3. A process for the preparation of a compound of formula (I) as defined in claim 1 or 2, by cyclization of a compound of formula (IV).

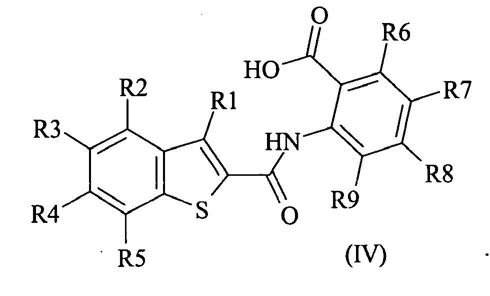

4. A process for the preparation of a compound of formula (I) according to claim 3, wherein the cyclization is done with acetic anhydride.

5. A process for the preparation of a compound of formula (I) according to claim 3 or 4, wherein the solvent is toluene, chlorobenzene, xylene or acetic anhydride.

6. A process for the preparation of a compound of formula (I) as defined in claim 1 or 2, wherein R1 is OC(O)R12 as defined in claim 1 or 2, by reacting a respective compound of formula (X), wherein R2, R3, R4, R5, R6, R7, R8 and R9 are defined as in claim 1 or 2, with a respective acid anhydride (R12C(O))20 or with a respective acid chloride R12C(O)C1, with R12 being as defined in claim 1 or 2.

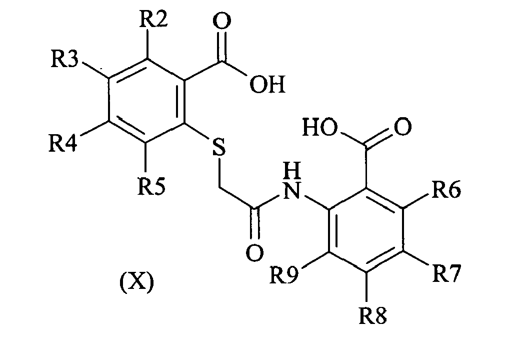

7. A process for the preparation of a compound of formula (I) according to claim 6, wherein the reaction is done in the presence of an inorganic or organic base.

8. A process for the preparation of a compound of formula (I) according to claim 6 or 7, wherein the compound of formula (X) is reacted with the respective acid chloride R12C(O)Cl in pyridine.

9. A process for the preparation of a compound of formula (I) according to claim 6 or 7, wherein the compound of formula (X) is reacted with the respective acid anhydride (R12C(O))20 in this respective acid anhydride.

10. Use of a compound of formula (I) as defined in claim 1 or 2, for the stabilization of an organic substrate.

11. Use of a compound of formula (I) according to claim 10, as light stabilizer.

12. Use of a compound of formula (I) according to claim 10 or 11, wherein the organic substrate is an organic polymer.

13. Use of a compound of formula (I) according to claim 12, wherein the organic polymer is a ethylene-vinylacetat copolymer, a polycarbonate or a polyester.
